# EUROPEAN PATENT SPECIFICATION

(11) **EP 2 486 943 B1**
(45) Date of publication and mention of the grant of the patent: **31.12.2014**
(21) Application number: 10822303.3
(22) Date of filing: 21.09.2010
(51) Int. Cl.: A61K 47/38, A61K 36/064, A61P 1/12

(54) **PHARMACEUTICAL COMPOSITION FOR USE IN THE PROPHYLAXIS AND TREATMENT OF INFECTIOUS AND NON-INFECTIOUS DIARRHOEA**
PHARMAZEUTISCHE ZUSAMMENSETZUNG ZUR VERWENDUNG IN DER PROPHYLAXE UND BEHANDLUNG INFEKTIÖSER UND NICHT INFEKTIÖSER DIARRHOE
COMPOSITION PHARMACEUTIQUE POUR L'UTILISATION DANS LA PRÉVENTION ET AU TRAITEMENT DE DIARRHÉES D'ORIGINE INFECTIEUSE OU NON-INFECTIEUSE

(30) Priority: 09.10.2009 RU 2009137408
(43) Date of publication of application: 15.08.2012
(73) Proprietor: Dikovskiy, Aleksander Vladimirovich, Moscow 123182 (RU)
(72) Inventor: RUDOI, Boris Anatolievich, Moscow 109518 (RU)
(74) Representative: Duncan, Garreth Andrew
(86) International application number: PCT/RU2010/000521
(87) International publication number: WO 2011/043695

(56) References cited:
- EP-A2- 0 149 579
- WO-A1-2007/117175
- RU-C1- 2 185 825
- DATABASE WPI Week 199532 Thomson Scientific, London, GB; AN 1995-244528 XP002711303, & RU 2 026 078 C1 (SORBTION TECHN CENTRE ENTERP) 10 January 1995 (1995-01-10)
- 'Spravochnik Vidal, Lekarstvennye preparaty v Rossii' ENTEROL vol. 14-E, 2008, page B-1262
- 'Spravochnik Vidal, Lekarstvennye preparaty v Rossii' FILTRUM STI vol. 14-E, 2008, page B- 1366
- FREXINOS J. ET AL.: 'Effect of carbolevure on intestinal fermentation induced by the ingetion of lactulose' INT. J. CLIN- PHARM. RES. vol. VIII, no. 6, 1988, pages 499 - 455, XP008153553

## Description

With regard to structure and disease incidence, acute infections of the intestinal tract (AIIT) rank second only to acute respiratory virus infections. Morbidity caused by AIIT is characterized by the absence of seasonal and age-specific changes in selectivity and development of unstable and short-term type-specific immunity, which assumes a repeat infection caused by another infectious agent during the limited period of time. Development of the new methods of treatment of AIIT and improvement of the existing ones take place simultaneously with accumulation of updated information derived from scientific research of the pathogenesis of infectious diseases. This creates the opportunities to develop new dosage forms and conduct clinical trials of the new drugs. This claim is intended to create a product which enables to abandon in some cases the traditional antibacterial agents used for treatment of infectious diseases, by strengthening the pathogenetic therapy in such a way that the patients will experience only minimal adverse effects.

The issues of etiopathogenetic therapy of AIIT remain topical owing to AIIT prevalence, increasingly severe clinical course of some nosological forms and occurrence of resistance to traditionally used etiotropic drugs. Extensive and not infrequently uncontrollable use of antibacterial agents is often not only insufficiently effective but is also accompanied by side effects and unfavorable consequences for the patient.

Among the acute infections of the intestinal tract, the so called "travelers' diarrhea" has attracted special attention lately. This is due to the increasing number of travelers and human migration. The travelers' diarrhea is a polyetiologic clinical syndrome which is characterized by increased stool frequency (more than three times a day) in the individuals leaving their countries or changing their climate zones and geographic regions. The vast majority of acute diarrhea cases are related to acute infections of the intestinal tract. However, the non-infectious factors can also be the cause of acute diarrhea, namely, faulty nutrition, diet overload, food incompatibilities, dietary crude fiber excess, allergic reactions to certain foods (mushrooms, shellfish, strawberries) or adverse drug effects.

The incidence of diarrhea, as well as other travelers' diseases, depends on the region, duration of stay, living environment (quality of water and nutrition particulars), as well as the type of activity. In the developing countries travelers' diarrhea clearly plays a dominant role as it affects 60% or more of arriving travelers. The travelers' diarrhea most frequently occurs in the Asian, African, Latin American and Middle Eastern countries, and less frequently in the Southern European and Caribbean countries (up to 20%). The USA, Canada, North European countries and Australia have the lowest diarrhea morbidity rate (less than 8%). Reportedly, diarrhea is sufficiently often found in citizens of the developing countries with poor sanitation and hygiene after they have arrived in the developed countries.

Different microorganisms such as bacteria viruses or protozoa are the causative agents of diarrhea. Other factors such as changes of diet and nutrition, living conditions, water salt content, alcohol abuse, non-customary food and changes of intestinal bacterial flora can be the cause of intestinal dysfunction after arrival in other countries or other climate zones and geographic regions.

The summary of infectious agents causing diarrhea in different regions (% cases) is presented in Table 1.

**Table 1**

| | | |
|---|---|---|
| Escherichia coli | | Up to 80 |
| of those: | enterotoxigenic | 40 - 60 |
| | enteropathogenic | 15 |
| | enteroinvasive | < 5 |
| Shigella organisms | | 10 |
| Salmonella organisms | | < 5 |
| Campylobacter organisms | | < 5 |
| Vibrio bacteria | | < 5 |
| Aeromonas species | | < 5 |
| Rotaviruses | | < 5 |
| Protozoa: | | |
| | Lamblia parasites | < 5 |
| | Entamoeba histolytica | < 5 |
| | Cryptosporidia | < 5 |

Up to 40% of diarrhea cases remain unidentified.

In different regions and during different seasons the ratio of causative pathogens can vary within 5 - 10% or more. Thus, as reported for some countries, the incidence rate of campylobacteriosis during the cold season of the year reaches up to a half of disease cases found, and the percentage of mixed infections (i.e., simultaneous presence of two or more causative agents) is 15 - 30%.

The main factors for transmission of infections are food, water and its derived ice, as well as beverages.

The risk of transmission is increased for travelers from the highly developed countries. They find themselves in the non-customary conditions especially when they did not visit tropical countries during the last 6 months, or are non-compliant with the dietary and hygienic precautions, or if they have gastrointestinal diseases (e.g., gastritis, Crohn's disease, ulcerative colitis or antacids intake) or immune deficiencies.

Despite the variety of pathogens causing travelers' diarrhea, there are several most typical clinical symptoms of the disease. The illness normally begins in 2-4, sometimes in 8-10 days, but no later than in two weeks after the arrival in a new country. Stool frequency varies from 3 - 5 to 6 - 15 times a day. There are moderately pronounced colicky intestinal pains accompanied by increased body temperature, chills, vomiting and arthralgia and myalgia as the manifestations of general toxicity syndrome. This is accompanied by a loss or absence of appetite for food.

Approximately 10% of patients are passing blood and/or mucous during defecation and manifesting febrile fever for more than three days and colicky pains in the lower abdomen. Such symptoms attest that the disease is getting worse, and this is generally associated with such causes of infection as shigella, enteroinvasive Escherichia coli or yersinia pathogens. With a certain level of disease severity, the following symptoms should be paid attention to: increased body temperature to 38.5°C or higher that lasts for three days or longer, blood in the stool, multiple episodes of vomiting and progressing dehydration.

The traditional (basic) treatment of travelers' diarrhea primarily includes starting rehydration as early as possible. Oral rehydration is not indicated during the gut-derived infectious-toxic shock, grade III-IV dehydration, unstable hemodynamics, pernicious vomiting, fluid loss of more than one liter/hr due to vomiting and diarrhea, diabetes mellitus or abnormal glucose absorption.

In the majority of cases the antibacterial treatment is not prescribed. Antibacterial drugs are indicated when the following symptoms are apparent: body temperature is increased to 38.5°C or higher and lasts for more than three days, presence of colitis syndrome, blood in the stool or progressing intoxication. In such cases the following fluoroquinolones are indicated: norfloxacin by 400 mg 2 times a day or ciprofloxacin by 500 mg 2 times a day. Typical duration of treatment is 3-5 days up to the moment of jugulation of clinical symptoms. Administration of the often recommended trimethoprim/sulfametoxasole and doxycycline should be treated with caution, as many microorganisms have developed significant resistance to these drugs, and serious adverse reactions to these medications are a growing concern. A widely prescribed anti-diarrheal medication loperamide can be administered for treatment of diarrheas caused by the non-invasive pathogens. However, it is hardly possible to make a satisfactory differential diagnosis at the early stages of disease. Recent recommendations on a wider administration of loperamide in combination with antibiotics can not be accepted unequivocally as well. It was noted above that antibiotics are indicated for treatment of travelers' diarrhea in about 10% of patients. Reportedly, bismuth subsalicylate is recommended for use in treatment of travelers' diarrhea.

In case of diarrhea caused by lamblia invasion ornidazole is preferable for treatment as lamblia has developed a significant resistance to metronidazole.

Diarrheas of different etiologies can be treated using the enterosorbents, for example, the lignin enterosorbents, such as "Filtrum-STI" tablets, or the mineral type sorbents, for example, the "Polysorb" or "Enterosgel" sorbents. Therefore, the use of enterosorbents is one of effective ways of treatment of not only infectious diarrheas but also of the abnormal dietary pattern-associated diarrheas (food disadaptation) which is reportedly found in nearly half of cases of travelers' diarrhea. Enterosorbents can be used as a supplementary therapy within the complex disintoxication treatment or as a monotherapy to treat the mild or moderately severe diarrheas.

Regardless of the etiology (viral or bacterial) or type of diarrhea (e.g., "invasive", "osmotic", etc.) the enterosorbents can be used as the sole etiotropic therapy of intestinal infections. In the beginning of disease the enterosorbents possessing the pathogenetic (disintoxicating, anti-diarrheal) and "etiotropic" effects can be used as they absorb and eliminate the pathogens [Novokshonov A.A., Mazankova L.N., Sokolova N.V. Pathogenetic justification of the optimal acute infections of the intestine treatment in children. // Pediatric infections, 2002, Nº 1, p. 32-37; Novokshonov A.A., Uchaikin V.F., Sokolova N.V et al. Biocenosis-preservation treatment of pediatric intestinal infections. // Pharmateca, 2004, 13 (90), p. 85-88]. Such enterosorbents primarily include the hydrolyzed lignin-based compounds (for example, Filtrum-STI lignin tablets), as well as the diosmectit-based mineral sorbents (such as Neosmectin or Smecta) or the alumosilicate gel compounds (e.g., Enterosgel). Filtrum is a trade name of the hydrolyzed lignin-based compound. The product is mostly released as tablets ("Filtrum-STI"). These enterosorbents can be used as the sole etiotropic therapy of mild and moderately severe diarrheas regardless of the type of diarrhea. With severe forms of diarrhea they can be used for treatment of osmotic and secretory types of diarrhea only starting from the first days of disease.

In addition to the above indicated enterosorbents, for complex treatment of diarrheas a number of sorbents with lower sorption potential are recommended, such as mineral sorbents (Attapulgite, Polysorb MP), microcrystalline cellulose-based sorbent (Microsorb-P), as well as some other types (Neointestopan, Reaban, Enterodes).

The abovementioned sorbents are administered in combination with other etiotropic medications. They can also be used as an alternative non-specific etiotropic therapy of mild forms of diarrhea. Enterosorbents exert rapid and pronounced detoxification and anti-diarrheal effects when used as the monotherapy or combination therapy, and significantly reduce the duration of acute period of disease.

It is worth noting that the effective doses of different sorbents used for treatment of diarrhea significantly differ from each other. Thus, the recommended for adults single dose of enterosgel is at least 15 g (daily dose is up to 45-50 g or 60-80 ml), dose of smectites is up to 9 g a day, and dose of polysorb is 9-12 g a day. The lignin-based compounds have the least single and daily doses, for example, Filtrum-STI adult dose is 6 tablets (about 2.4 g lignin) a day to treat mild diarrheas, and up to 8 tablets (about 3 g) a day given in 3-4 divided doses of 2 tablets each, or about 0.8 g of sorbent taken as a single dose. At the same time, the laboratory data on sorption activity of the most effective sorbents, such as smectites, lignins or pectins, demonstrate that in *in vitro* conditions their ability to absorb various toxic substances or pathogenic bacteria is quite comparable.

The pathogenetic mechanisms of enterosorbents' positive effects during diarrhea treatment are more complex than it can be described in purely physical terms for sorption of toxins and viruses on the surface of sorbent particles. Apart from direct binding of pathogenic microorganisms and toxins, enterosorbents exert their effects by employing some additional mechanisms, namely:
- adsorption mediated by secondary sorption effects (adsorption by polymeric molecules bound to sorbent particles);
- direct and indirect binding of fluid (owing to absorption capture, and through the binding of osmotically active free fatty acids, which is of particular importance in osmotic diarrheas);
- cytoprotector (protective) effect on the epithelial cells of intestinal mucous membrane (coating effect);
- besides, because the sorbents represent crude, non-digestible components of the food bolus, they induce complex specific reactions leading to the inhibition of peristaltic activity.
It should be noted that, unlike the group of mineral sorbents, purified cellulose-based sorbents or synthetic polymer molecules of Enterodes type, the medicinal lignin-based enterosorbents are derived from the natural plant raw material (wood pulp) which contains the enormous amount of various biologically active compounds of different groups (antioxidants, tannins, plant hormone-like substances, plant poly- and oligosaccharides possessing the immunostimulating effects, plant compounds with marked antibacterial and antiviral activity and substances having the cytoprotector activity). Production of medicinal lignins makes use of the technology of processing of raw plant material which enables to obtain products with high sorption ability and retaining at the same time the vast amount of these useful natural components. The latter provide significantly higher therapeutic effects of lignins while keeping their doses low. The mineral sorbents, synthetic polymeric sorbents and even the highly purified natural cellulose-based sorbents, as well as activated charcoals produced by burning the basic charcoal, are lacking such useful components.

However, even such highly effective enterosorbents as medicinal lignins can not be reasonably fast and efficiently protective at the same time, when the intestinal infections are caused by microbial agents of different types. The shortage of enterosorbents in treatment of diarrheas is that their use is restricted only to cases of mild or moderately severe illness, also, rather extended time periods before the results of treatment become obvious. For treatment of more severe diarrheas, particularly those caused by highly pathogenic bacteria, the antibiotics or other antimicrobials are used in order to suppress the intestinal growth of pathogenic microorganisms.

However, the therapeutic potential of antibiotics is also limited. Administration of synthetic antibacterial and antimicrobial agents for treatment of travelers' diarrhea often brings not so much positive but rather negative effect, as up to 40-50% of cases of travelers' diarrhea are caused by either non-bacterial pathogens (for example, by amoebas or other protozoa) or non-infectious factors (abnormal dietary pattern or food disadaptation, inclusive of water disadaptation). For this reason the administration of antibacterial or other antimicrobial agents is justified only when the infectious etiology of diarrhea is confirmed, and the disease has a severe course.

There is also a method of treatment of different types of diarrheas by oral administration of live probiotic bacteria (for example, probifor, bifiform, sporobacterin, polybacterin, etc.), inclusive of preparation containing live culture of yeasts Saccharomyces cerevisiae var. boulardii (trade name "Enterol") (see, for example, Use of saccharomyces yeasts for the manufacture of a medicament for preventing or treating pseudomembranous colitis. EP 149579 (A2) 1985-07-24 HUBLOT BERNARD; LEVY RENE HANANIA, BIOCODEX LAB [FR], A61K36/064, A61K36/06, (IPC1-7): A61K35/72, also: Treating weight loss in patients suffering from inflammatory bowel diseases using S. boulardii EP1693064 (A1) 2006-08-23 HUBLOT BERNARD [FR]; GROUX HERVE [FR]; BERNASCONI PAUL [FR]; LEVY RENE [US]; LE GUERN MARIE-EMMANUELLE [FR] BIOCODEX [FR] A61K36/064; A61P1/00; A61K36/06; A61P1/00.

Firstly, *Saccharomyces boulardii* exerts some antimicrobial effect against the intestinal pathogenic and conditionally pathogenic microorganisms and yeasts (*Clostridium difficile, Candida krusei, Candida pseudotropicalis, Candida albicans, Klebsiella spp., Proteus spp., Pseudomonas aeruginosa, Staphylococcus auerus, Escherichia coli, Salmonella typhi, Shigella spp., Entamoeba histolytica, Gardia lambliae, Vibrio cholerae,* etc.). It should be underscored that the antagonistic action of this type of yeasts is associated with the release of a number of antimicrobial factors specifically in the process of yeast life activity cycle. The antagonistic action of the preparation is abolished when *Saccharomyces boulardii* cells are killed by heating. This is the only example of the *in vivo* antimicrobial antagonism exerted by the microorganism used for therapeutic purposes, and which is not a part of the normal intestinal flora, but is able to pass through the gastrointestinal tract in significant quantities and without losing its viability.

The other mechanism of *Saccharomyces boulardii* action is the *in vitro* proven anti-secretory effect owing to reduced production of adenylate cyclase and ensuing enterotoxin-induced decrease of salt and water secretion. It is established that a 120 kDa protein is responsible for this mechanism of action when diarrhea is caused by *Vibrio cholerae* or an enterotoxigenic *Escherichia coli. Saccharomyces boulardii* also exerts direct antitoxic effect thanks to the yeast- produced 54 kDa trypsin-like protease which is able to specifically recognize and cleave toxins (toxin A of *Clostridium difficile,* in particular) and receptors on the surface of enterocyte microvilli which constitute the binding site for the toxin.

*Saccharomyces boulardii* are naturally resistant to all antibacterial antibiotics, which is not characteristic for bacterial microorganisms. Unlike fluconazole, nystatin is not absorbed from gastrointestinal tract, and its administration in man brings complete elimination of yeasts from the fecal matter. Oral administration of Enterol® in 4-6 h after fluconazole administration does not affect the viability of *Saccharomyces boulardii* in the gut. Hydrochloric acid in the gastric juice has no effect on *Saccharomyces boulardii,* they are not digested and can be found as live cells in all parts of the gastrointestinal system. As *Saccharomyces boulardii* can survive while not colonizing the human colon, daily administration of yeast preparation is required to maintain a steady concentration of > 10⁶ colonies/g in cecal content.

The drawback of the live yeast *Saccharomyces boulardii* preparations is a significant variation of their efficacy against different pathogenic and conditionally pathogenic microorganisms and yeasts present in the gut, as well as some other pathogens, also, the insufficiently rapid efficacy of treatment, not enough effective antimicrobial and antitoxic effect and their inability to bind and actively excrete the already accumulated in the gut toxic products. As a result this may allow continuous absorption of such toxic products leading to chronic intoxication even at the backdrop of administration of *Saccharomyces boulardii* preparations.

For prophylaxis and treatment of severe forms of diarrhea some combination preparations were introduced into clinical practice. Such preparations combine probiotic organisms inclusive of yeast cultures, and the substances with known sorption abilities. The example of such preparation is Bifidumbacterin-forte containing freeze-dried live bacterial strain of *Bifidobacterium bifidum* Nº 1 and possessing the antagonistic activity against pathogens. At least 5 x 10⁷ CFU of bifidobacterium is absorbed onto the activated charcoal particles. *Pharmacological action:* normalization of intestinal microflora, improvement of health of digestive system and immune modulation. Bifidumbacterin-forte is a broad-spectrum antagonist of pathogenic and conditionally pathogenic microorganisms, prevents their adhesion to intestinal mucous membrane, helps to restore the microbiocenosis of gastrointestinal tract (GIT), activates parietal digestion and synthesis of vitamins and amino acids and increases non-specific resistance to pathogens. *Indications:* dysbacteriosis inclusive of that in GIT diseases (peptic ulcer disease [gastric or duodenal ulcers], pancreatitis, cholecystitis, hepatitis, etc.), respiratory diseases (acute respiratory viral infections, bronchitis, pneumonias) and diseases of urogenital tract, allergic diseases, administration of antibacterial drugs (including antibiotics), hormones and NSAIDs, acute intestinal infections of the established (shigellosis, salmonellosis, staphylococcal enterocolitis, rotavirus infection) and non-established etiology, food toxicoinfection, diarrhea at the backdrop of long-term antibacterial therapy, malabsorption syndrome, chronic constipation, correction of microbiocenosis and prophylaxis of purulent-septic diseases before and after surgery on the intestine, liver or pancreas).

The drawbacks of Bifidumbacterin-forte are its insufficient antagonistic activity against highly pathogenic strains of Escherichia coli and staphylococci, absence of cytoprotective effect on the enterocytes of intestinal mucous membrane, low antiviral activity, irritation of intestinal mucosa by charcoal particles, insufficient sorption activity of charcoal enterosorbent with regard to high molecular weight toxins and pathogenic bacteria and viruses, thus making Bifidumbacterin-forte a not sufficient enough medication for treatment of AIIT, and travelers' diarrhea in particular.

There is a combination medication (trade name Carbolevure) intended for prophylaxis and treatment of diarrheas, which contains a sorbent (activated charcoal) and live Saccharomyces cerevisiae yeast cells (Frexinos J., Bloom M., Milinkevitch D. Effect of Carbolevure on intestinal fermentation induced by the ingestion of lactulose. / Int. J. Clin. Pharmacol. Res. 1988, 8 (6): 449-55). The drawbacks of this medication are its weak therapeutic efficacy, which is due to low ability of activated charcoal to absorb microbial cells, irritation of gastrointestinal mucosa by charcoal particles and the use of the yeast strain having low antagonistic activity against pathogenic microorganisms.

Also, there are preparations which include the useful yeast strains and low processed crude food fibers possessing sorption abilities, such as wheat or rye offal. For example, method of producing food supplement (RU Nº 2233320) stipulates the propagation and immobilization of Saccharomycetaeceae biomass on a carrier. The preparation represents food supplement composed of the *Saccharomyces cerevisiae* cell components immobilized onto a porous carrier of plant origin. The carrier is a mixture of native and extruded offal of cereal crops, and has a porous surface area of at least 2.0 m²/g. The concentration of the *Saccharomyces* cells is at least 10⁹-10¹² cells/g. The preparation contains (% by weight) lipids (2-5), carbohydrates (25-50), proteins, vitamins, macroelement and food fibers. The proteins include 10 essential amino acids in quantity of 30.0 - 75.0 % by weight of total protein. The vitamins are B, B₁, B₂, B₃, B₆, PP and C. The macroelements include phosphorus, potassium, sodium, magnesium and calcium. Soluble food fibers make at least 45 % by weight, and the insoluble food fibers - at least 35 % by weight. This enables to obtain the food supplement and medicinal product having the marked probiotic effect, high bifido- and lactogenic effects, as well as good sorption ability.

There is a preparation containing sterile offal, its grown microbial cells of the *Saccharomyces cerevisiae* strain (vini) VKMP Y-511, biologically active substances and microelements. The preparation contains inactivated microbial cells of the *Saccharomyces cerevisiae* strain (vini) VKMP Y-511 in quantity of 2.55 x 10¹⁰ - 2.65 x 10¹⁰ microbial cells per 1 g offal. The offal is wheat offal of alimentary grade, sterile, red-yellow, with no odor and microelement content of 4.8 - 5.5%. The preparation contains the following microelements: calcium (0.10 - 0.15 wt. %), phosphorus (1.00 - 1.40 % by weight), sodium (0.08 - 0.10 % by weight), zinc (106-109 x 10⁻⁶ % by weight) and iron (114 - 118 x 10⁻⁶ % by weight). Production method includes culturing of the *Saccharomyces cerevisiae* strain (vini) VKMP Y-511 in offal with its pre-dampening with water and subsequent heat treatment followed by sterilization of the finished product. The offal is dampened before culturing the organism using the odorless water which turbidity is below 0.1, pH is 8.7, containing no chlorine, with permanganate oxidability (in mgO/dm³) of no more than 1.6 mg/dm³ and water hardness of 1.5 mg/dm³ and total mineralization no more then 384 mg/dm³. Heat treatment during culturing the organism is carried out by evenly heating up the total volume of culture medium (RU Nº 2206330).

The drawback of the above compositions of sorbents and yeasts is the introduction in the production process of a stage of inactivation of live yeast cells which are losing a significant fraction of their antagonistic activity against pathogenic microorganisms at that.

Besides, wheat or rye offal has an insignificant sorption activity against pathogenic microorganisms during its passage through the human intestine.

There is a known method of prophylaxis and treatment of intoxications due to intestinal dysbacteriosis, which is an oral administration of live baker's yeast cells and excipient components (transporting, sorption, nutritional, etc.) represented by laminaria, garlic (dry powder), polyvitamins and microelements (standard set), ascorbic xyloma and wheat offal. The components are present in the following mass ratios (parts):
*Saccharomyces cerevisiae* yeasts - 10 - 12,
Laminaria - 3 - 5,
Garlic (dry powder) - 3 - 5,
Polyvitamins and microelements (standard set) - 0.1 - 0.2,
Ascorbic acid - 1 - 2,
Wheat offal - up to 100.

For prophylactic purposes 10-15 g of the mixture is administered daily for 7-14 days, and for treatment purposes 15-20 g of the mixture is administered daily for 3-7 days until the symptoms of intoxication have gone (RU Nº 97113383/14).

The drawbacks of this composition of sorbents and yeasts are the low antagonistic activity of baker's yeasts against many pathogenic microorganisms causing diarrhea, and not sufficiently high ability to absorb pathogenic microorganisms of the offal.

Technical objective of this invention is to create a universal and effective pharmaceutical composition for prophylaxis and treatment of infectious and non-infectious diarrheas and to expand the arsenal of pharmaceutical compositions for prophylaxis and treatment of infectious and non-infectious diarrheas.

In order to achieve this technical objective we need to solve a number of tasks including the expansion of the range of susceptible to treatment diarrhea pathogens (unified treatment for various pathogen types, see Table 1), shortening the treatment time and reduction of variance in treatment time with regard to many other pathogens causing food-borne toxicoinfections and travelers' diarrhea, reduction of severity of diarrheal symptoms at the expense of the etiopathogenetic effect of the indicated yeast strain leading to increased antagonistic activity and production of immunostimulating factors, activation of absorption of toxic substances by lignin and normalization of intestinal motor function.

The present invention is defined by the claims.

The essence of invention regarding the pharmaceutical composition for use in prophylaxis and treatment of infectious and non-infectious diarrheas, is that it includes the hydrolyzed medicinal lignin and live cells of *Saccharomyces boulardii* yeast strain taken at 10⁹ - 5 x 10¹⁰ live yeast cells per 1 gram of hydrolyzed lignin. The pharmaceutical composition is preferably prepared by the uniform mixing of lyophilized powder of cells of *Saccharomyces boulardii* yeast strain with the powder of medicinal lignin.

The claimed invention is intended for prophylaxis and treatment of diarrheas and acute intestinal infections, and is based on using for this purpose the combination of the most effective of all known enterosorbents (hydrolyzed lignin) and live yeast cells with the highest antagonistic activity, the *Saccharomyces boulardii* yeast strain, both taken in effective therapeutic quantities. Their combination leads to mutual potentiation of useful effects of both components (synergistic activity), i.e., the antagonistic activity of live yeast cells in combination with lignin sorption ability, as well as its ability to normalize the intestinal motor activity and cause additional physiological effects to secure the achievement of a non-obvious effect. For example, given the approximately equal effectiveness of each of the two components with regard to reduction of duration of symptoms of general intoxication (apathy, vomiting, nausea, diarrhea, etc.), their composition gives a statistically significant further reduction of duration and severity of symptoms (by 1-3 days for different symptoms) depending on the type of pathogen causing food intoxication, diarrhea and concomitant symptoms. The important role belongs to the trophic effect of pharmaceutical composition owing to release of polyamines (spermine and spermidine) by *Saccharomyces boulardii.* Polyamines enhance glucose absorption by enterocytes via the stimulation of membrane transport systems. Disaccharidase activities of *Saccharomyces boulardii* is directly linked to the trophic effect. Increased enzymatic activity of disaccharidases (sucrose-alpha-glucosidase, lactase and maltase in epithelial intestinal cells) promotes a significant improvement of carbohydrate absorption in diarrhea and most effectively prevents intestinal fermentation in the course of treatment with the claimed pharmaceutical composition. Therefore, this pharmaceutical composition can restore the metabolic activity of intestinal mucosa in the shortest possible time.

At the same time it gives a powerful boost to the local immune defense by activating complement system and increasing the IgA and other immunoglobulin secretion in the enterocytes' crypts and intestinal mucosa villi. The immunostimulating effect is promoted by glucan component of the cell wall of *Saccharomyces.*

The efficacy of this pharmaceutical combination significantly exceeds that of all known similar preparations while showing the highest rapidity and universality of action.

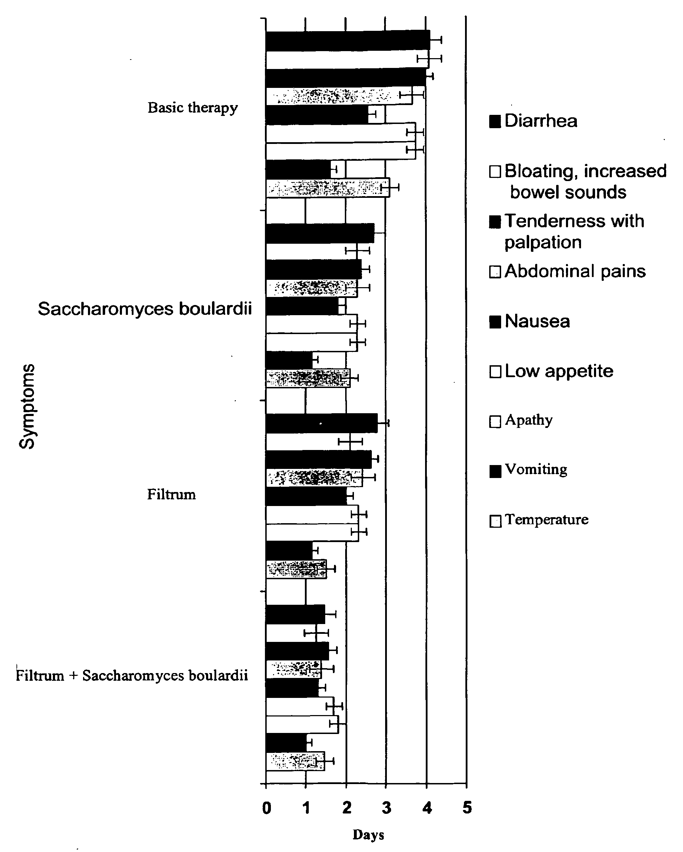

The chart (Figure 1) depicts the duration of symptoms during different types of treatment used in clinical trials. The information on the preparations compared is given on the left-hand side, and the number of days of duration of symptoms is given below the chart. For each of compared preparations a group of temporal histograms of controlled symptoms is given. The temporal histograms showing the duration of each symptom are arranged one after another within each group and in the same order as their corresponding symptoms are listed in the right column.

The following studies on clinical efficacy of the claimed pharmaceutical composition used as a component of complex therapy of acute intestinal infections in adults, as well as for confirmation of achievement of technical result of the use of pharmaceutical composition which includes "Filtrum" preparation (hydrolyzed medicinal lignin) and live cells of *Saccharomyces boulardii* yeast strain taken at 10⁹ - 5 x 10¹⁰ live yeast cells per 1 gram of hydrolyzed lignin, have been carried out.

Specifically, the claimed pharmaceutical composition is supposed to contain the powder or granules of hydrolyzed medicinal lignin and its bound live, e.g., freeze-dried, i.e., staying in an anabiotic state, cells of *Saccharomyces boulardii* yeast strain. It is a good practice to prepare the composition by mixing the components, namely, by adding to medicinal lignin powder of the freeze-dried (dried from a frozen state in a deep vacuum) powder of live (or to be more precise, viable or staying in an anabiotic state) cells of *Saccharomyces* yeast strain. Upon hydration of the mixture the dried yeast cells recover their viability and start producing active substances.

However, once in the gut (after their oral administration) the *Saccharomyces boulardii* yeasts (unlike the brewer's or baker's yeasts) can not actively propagate because of their high concentration on the sorbent and conditions of the intestinal medium (pH reaction, oxidation-reduction potential, etc.) non-optimal for their growth. For these reasons yeasts can not cause further diarrhea complications due to active production of gas or low-molecular organic acids.

The clinical efficacy study of the claimed pharmaceutical composition was carried out at the Infectious Clinical Hospital Nº 3 of Moscow health department (Chief physician - L.I. Lazutkina).

Sixty-eight patients aged from 17 to 72 years, with food toxicoinfections of moderate severity were under clinical observation. The diagnosis was established based on typical clinico-epidemiological data, and was verified by bacteriological and serological methods which allowed ruling out the presence of dysentery and salmonellosis pathogens.

Patients were randomized into 4 groups using the single-blind method.
Group 1 - in addition to basic therapy, patients received the claimed pharmaceutical composition containing "Filtrum" (2 tablets 3 times a day) + enterol (Saccharomyces boulardii) (live cells of *Saccharomyces boulardii* yeast strain taken at 10⁹ - 5 x 10¹⁰ live yeast cells per 1 gram of hydrolyzed lignin) 3 times a day (20 subjects);
Group 2 - in addition to basic therapy, patients received "Filtrum" (2 tablets 3 times a day) (16 subjects);
Group 3 - in addition to basic therapy, patients received enterol (Saccharomyces boulardii) by 1 capsule 3 times a day (16 subjects);
Group 4 (reference group) - patients received basic therapy only (16 subjects).

Basic treatment included rehydration therapy using crystalloid polyionic parenteral ("Acesol", Chlosol") and oral ("Rehydron") solutions, enzymes (mezym-forte, panzim-forte, festal), antispasmodics (no-spa, platyphylline), calcium gluconate and polyvitamins.

To assess clinical efficacy of different types of treatment the duration of the following symptoms of food toxicoinfections was taken into account:
- body temperature above 37°C;
- vomiting;
- atony;
- low appetite;
- nausea;
- abdominal pains;
- tenderness with abdominal palpation;
- bloating, increased bowel sounds;
- diarrhea.

Comparative evaluation of duration of the main clinical symptoms of food toxicoinfections in patients from different groups was carried out using traditional statistical methods. Student's t-test was used for evaluation of degree of statistical significance (p-value) of differences between the parameters.

### RESULTS

Table 2 presents the duration of the main clinical symptoms in patients with food toxicoinfections (days, M ± m).

**Table 2**

| Clinical symptoms | Patient groups | | | |
|---|---|---|---|---|
| | "Filtrum" + Saccharomyces boulardii | "Filtrum" | Saccharomyces boulardii | Basic treatment |
| Body temperature above 37°C | 1.47 ± 0.17** | 1.5 ± 0.25** | 2.1 ± 0.29* | 3.11 ± 0.33 |
| Vomiting | 1.0 | 1.14 ± 0.15 | 1.14 ± 0.15 | 1.62 ± 0.28 |
| atony | 1.8 ± 0.12** | 2.33 ± 0.25* | 2.3 ± 0.16* | 3.75 ± 0.44 |
| low appetite | 1.7 ± 0.13** | 2.33 ± 0.18* | 2.3 ± 0.16* | 3.75 ± 0.44 |
| Nausea | 1.29 ± 0.15* | 2.0 ± 0.16 | 1.8 ± 0.21 | 2.56 ± 0.4 |
| abdominal pains | 1.39 ± 0.15** | 2.43 ± 0.32 | 2.3 ± 0.16 | 3.67 ± 0.59 |
| tenderness with abdominal palpation | 1.56 ± 0.15** | 2.62 ± 0.26 | 2.4 ± 0.17* | 4.0 ± 0.58 |
| bloating, increased bowel sounds | 1.26 ± 0.15** | 2.12 ± 0.29* | 2.3 ± 0.16* | 4.1 ± 0.65 |
| Diarrhea | 1.45 ± 0.12*** | 2.78 ± 0.34* | 2.7 ± 0.31* | 4.11 ± 0.33 |

| | | | | |
|---|---|---|---|---|
| *Note:* significance of differences between parameters as compared to the patient group receiving basic treatment: * p < 0.05; ** p < 0.01; *** p < 0.001. | | | | |

In the patient groups (Table 2) receiving, in addition to basic treatment, either the pharmaceutical composition "Filtrum" + live cells of *Saccharomyces boulardii* yeast strain taken at 10⁹ live yeast cells per 1 gram of hydrolyzed lignin, or "Filtrum", or enterol, the duration of such symptoms of intoxication as increased body temperature, atony, low appetite was significantly lower (p < 0.05; p < 0.01) as compared to the patient group receiving basic treatment only. Only in the patient group receiving additionally "Filtrum" *+ Saccharomyces boulardii* the duration of nausea period was significantly shorter against that in the patient group receiving only basic treatment (p < 0.05). Administration of "Filtrum" *+ Saccharomyces boulardii* significantly reduced the duration of abdominal pains and tenderness with abdominal palpation (p < 0.01) as compared to the basic therapy group. No significant differences in the duration of pain syndrome were found between the "Filtrum" group and basic therapy group. Administration of *Saccharomyces boulardii* on its own significantly shortened the period of tenderness with abdominal palpation (p < 0.05) as compared to basic treatment group, but without any significant effect on the duration of abdominal pain. The duration of bloating and increased bowel sounds' periods or diarrhea in patients treated with "Filtrum" or *Saccharomyces boulardii* was significantly shorter against that in the patient group receiving only basic treatment (* p < 0.05; ** p < 0.01; *** p < 0.001) but longer than in patients treated with composition of Filtrum" + *Saccharomyces boulardii.*

Therefore, in contrast to separate administration of Filtrum" or *Saccharomyces boulardii,* administration of composition of "Filtrum" + *Saccharomyces boulardii* was accompanied by statistically significant reduction of duration of all clinical symptoms of food intoxications as compared to those in patients receiving only basic treatment.

We also carried out comparative studies on the duration of clinical symptoms in patients treated by a combination of medicinal lignin ("Filtrum" + *Saccharomyces boulardii*) or each of components on its own ("Filtrum" or *Saccharomyces boulardii*) (Tables 3 and 4).

Table 3 presents comparative evaluation of duration of the main clinical symptoms in patients with food toxicoinfections treated by a combination of "Filtrum" + *Saccharomyces boulardii* or only "Filtrum" (days, M ± m).

**Table 3**

| Clinical symptoms | Patient groups | | p |
|---|---|---|---|
| | "Filtrum" + Saccharomyces boulardii | "Filtrum" | |
| body temperature above 37°C | 1.47 ± 0.17 | 1.5 ± 0.25 | p > 0.05 |
| vomiting | 1.0 | 1.14 ± 0.15 | |
| atony | 1.8 ± 0.12 | 2.33 ± 0.25 | p > 0.05 |
| low appetite | 1.7 ± 0.13 | 2.33 ± 0.18 | p < 0.05 |
| nausea | 1.29 ± 0.15 | 2.0 ± 0.16 | p < 0.05 |
| abdominal pains | 1.39 ± 0.15 | 2.43 ± 0.32 | p < 0.05 |
| tenderness with abdominal palpation | 1.56 ± 0.15 | 2.62 ± 0.26 | p < 0.01 |
| bloating, increased bowel sounds | 1.26 ± 0.15 | 2.12 ± 0.29 | p < 0.05 |
| diarrhea | 1.45 ± 0.12 | 2.78 ± 0.34 | p<0.01 |

Administration of combination of "Filtrum" + Saccharomyces boulardii (live cells of *Saccharomyces boulardii* yeast strain taken at 2 x 10¹⁰ live yeast cells per 1 gram of hydrolyzed lignin) was accompanied by significantly faster resolution of symptoms of gastrointestinal syndrome (abdominal pains, tenderness with abdominal palpation, bloating, increased bowel sounds or diarrhea) (p < 0.05; p < 0.01) as compared to administration of "Filtrum" only. The duration of such symptoms of intoxication as increased body temperature or atony was not significantly different between the two groups. However, nausea resolved significantly faster after administration of "Filtrum" + Saccharomyces boulardii as compared to administration of "Filtrum" only (p < 0.05), and the appetite was restored significantly faster (p < 0.05).

Table 4 presents comparative evaluation of duration of the main clinical symptoms in patients with food toxicoinfections treated by a combination of "Filtrum" + Saccharomyces boulardii or Saccharomyces boulardii only (days, M ± m).

**Table 4**

| Clinical symptoms | Patient groups | | p |
|---|---|---|---|
| | "Filtrum"" + Saccharomyces boulardii | Saccharomyces boulardii | |
| body temperature above 37°C | 1.47 ± 0.17 | 2.1 ± 0.29 | p > 0.05 |
| vomiting | 1.0 | 1.14 ± 0.15 | |
| atony | 1.8 ± 0.12 | 2.3 ± 0.16 | p < 0.05 |
| low appetite | 1.7 ± 0.13 | 2.3 ± 0.16 | p < 0.05 |
| Nausea | 1.29 ± 0.15 | 1.8 ± 0.21 | p >0.05 |
| abdominal pains | 1.39 ± 0.15 | 2.3 ± 0.16 | p < 0.01 |
| tenderness with abdominal palpation | 1.56 ± 0.15 | 2.4 ± 0.17 | p < 0.01 |
| bloating, increased bowel sounds | 1.26 ± 0.15 | 2.3 ± 0.16 | p < 0.01 |
| diarrhea | 1.45 ± 0.12 | 2.7 ± 0.31 | p < 0.01 |

A similar trend was found upon comparative analysis of duration of clinical symptoms of food intoxications in patients treated with the combination of "Filtrum" + Saccharomyces boulardii and Saccharomyces boulardii only. Administration of combination of "Filtrum" + Saccharomyces boulardii was accompanied by significantly faster resolution of symptoms of gastrointestinal syndrome (abdominal pains, tenderness with abdominal palpation, bloating, increased bowel sounds or diarrhea) (p < 0.01) as compared to administration of Saccharomyces boulardii only. There were no significant differences in terms of duration of increased body temperature or nausea between the two groups. However, the atony and appetite loss have resolved significantly faster in patients treated with the combination of "Filtrum" + Saccharomyces boulardii as compared to patients treated with Saccharomyces boulardii only (p < 0.05).

The claimed pharmaceutical composition "Filtrum" + Saccharomyces boulardii was studied in 119 pediatric patients aged from 2 months to 13 years suffering from the AIIT, mainly mild or moderately severe forms, inclusive of 38 patients with viral AIIT (rotaviruses, Norwalk virus, toroviruses). Sixty-one patients received the pharmaceutical composition "Filtrum" + Saccharomyces boulardii, and 58 patients (control group) received only basic therapy.

Administration of the pharmaceutical composition "Filtrum" + Saccharomyces boulardii led to faster (by 1-3 days) jugulation of fever, tenderness with abdominal palpation, diarrhea (with stool frequency up to 5 times a day) and mucous in stool (tenderness with abdominal palpation, in particular, disappeared nearly 2 days earlier). Stool consistence was normalized, and undigested food residues disappeared from the stool by 2 - 2.5 days earlier in the composition-treated subjects. In 70% of children fever was jugulated during the first 2 days of treatment. Flatulence symptoms disappeared by day 3 of combination treatment in nearly 87% of patients. The comparison of treatment results in different treatment subgroups has shown that the pharmaceutical composition "Filtrum" + Saccharomyces boulardii was most efficient in treatment of viral diarrheas as the diarrheal symptoms and tenderness with abdominal palpation have disappeared significantly faster in this treatment group.

Table 5 presents the evaluation of duration of the main clinical symptoms in pediatric patients with AIIT treated by the pharmaceutical composition "Filtrum" + Saccharomyces boulardii (live cells of *Saccharomyces boulardii* yeast strain taken at 5 x 10¹⁰ live yeast cells per 1 gram of hydrolyzed lignin) (days, M ± m).

**Table 5**

| Symptoms | Mean duration of symptoms | | |
|---|---|---|---|
| | Salmonellosis (n = 9) | Viral diarrheas (n = 9) | Gastroenteritis of unknown etiology (n = 23) |
| Fever | 1.4 ± 0.25 | 1.33 ± 0.33 | 2.33 ± 0.33 |
| Tenderness with palpation | 3.3 ± 0.44 | 2.0 ± 0.21 | 2.33 ± 0.38 |
| Diarrhea (stool up to 5 times a day) | 4.47 ± 0.38 | 3.44 ± 0.29 | 3.29 ± 0.33 |
| Mucous in stool | 2.6 ± 0.41 | 2.80 ± 0.39 | 2.20 ± 0.33 |

This study of the clinical efficacy of pharmaceutical composition of the enterosorbent-medicinal lignin ("Filtrum") + Saccharomyces boulardii in comparison with treatment by "Filtrum" or Saccharomyces boulardii on their own, enables to draw the following conclusions.

The administration of combined preparation "Filtrum" + Saccharomyces boulardii as a supplement to basic therapy results in statistically significant reduction of duration of all clinical symptoms of food toxicoinfections as compared to that in patients receiving basic therapy only. Such treatment effect was not observed in patients treated by "Filtrum" or Saccharomyces boulardii on their own. Administration of pharmaceutical composition of "Filtrum" + Saccharomyces boulardii results in reduced duration of symptoms of gastrointestinal syndrome (abdominal pains, tenderness with abdominal palpation, bloating, increased bowel sounds or diarrhea) in patients with food toxicoinfections, and the recovery is significantly faster than that during treatment by each of the two components on their own (see chart). One of the important points which determine the results of this claim is a synergy between the medicinal lignin and live yeast cells, not only in terms of combination of absorption and binding of toxins and bad bacteria but also owing to the presence in yeasts and medicinal lignin of particular chemical substances which cooperate in effectively inhibiting the growth of pathogenic microorganisms (including viruses), as well as additional protection of intestinal cells due to enhanced antioxidant effect and stimulation of epithelium growth and regeneration via the reinforced formation of complex gel-like structures along the intestinal wall and acceleration of humoral immune response accompanied by production of hormone-like substances (e.g., regulators of digestion) and signal molecules by lymphoid and macrophage cells of the reticuloendothelial system. The variance of treatment efficacy for various pathogens causing food toxicoinfections, travelers' diarrhea and concomitant symptoms was virtually absent.

No adverse effects in the form of allergic conditions, drug-induced disease, deterioration in wellbeing or aggravation of clinical signs of the main disease have been found in patients during administration of the pharmaceutical composition of medicinal lignin and Saccharomyces boulardii.

The claimed preparation (pharmaceutical composition) is for use in the treatment of travelers' diarrhea which may be used on the road when making a diagnosis or finding the cause of diarrhea is difficult to do, as well as when in urgent need of taking some effective drug (and a doctor is not available) to reduce the frequency of lavatory visits. The abovementioned pharmaceutical composition can have a marked antimicrobial and antiviral effect, or at least can normalize the motor function of the intestines (in case of non-infectious diarrhea).

Therefore, we have created a universal and effective pharmaceutical composition for prophylaxis and treatment of infectious and non-infectious diarrheas. This expands the arsenal of pharmaceutical compositions used for prophylaxis and treatment of infectious and non-infectious diarrheas.

A formulation has been developed that enables suppression of a wide spectrum of causative agents of diarrheas and food toxicoinfections, reduction of variations in treatment effect and shortening of treatment time.

## Claims

1. Pharmaceutical composition for use in prophylaxis and treatment of infectious and non-infectious diarrheas, which comprises hydrolyzed medicinal lignin and live cells of *Saccharomyces boulardii* yeast strain taken at 10⁹ - 5 x 10¹⁰ live yeast cells per 1 gram of hydrolyzed lignin.

2. The composition for use according to claim 1, **characterized in that** it is prepared by the even mixing of freeze-dried powder of *Saccharomyces boulardii* yeast cells with the powder of medicinal lignin.

3. The composition for use according to any of claims 1 or 2, **characterized in that** the medicinal lignin is present in the form of powder or of granules.

4. The composition for use according to any of claims 1 to 3, **characterized in that** said composition is hydrated and that the dried yeast cells recover their viability and start producing active substances.

5. The composition for use according to any of claims 1 to 4, **characterized in that** it is used for treatment of travelers' diarrhea.

6. Use of a composition comprising hydrolyzed medicinal lignin and live cells of *Saccharomyces boulardii* yeast strain taken at 10⁹ - 5 x 10¹⁰ live yeast cells per 1 gram of hydrolyzed lignin for the manufacture of a medicament for prophylaxis and treatment of infectious and non-infectious diarrheas.

## Patentansprüche

1. Pharmazeutische Zusammensetzung zur Verwendung in der Prophylaxe und Behandlung von infektiösen und nichtinfektiösen Diarrhöen, die hydrolysiertes medizinisches Lignin und lebende Zellen des Hefestamms *Saccharomyces boulardii* in einer Menge von 10⁹ - 5 x 10¹⁰ lebenden Hefezellen pro Gramm hydrolysiertes Lignin umfasst.

2. Zusammensetzung zur Verwendung nach Anspruch 1, **dadurch gekennzeichnet, dass** sie durch gleichmäßiges Mischen von gefriergetrocknetem Pulver von *Saccharomyces-boulardii*-Hefezellen mit dem medizinischen Ligninpulver hergestellt wird.

3. Zusammensetzung zur Verwendung nach einem der Ansprüche 1 oder 2, **dadurch gekennzeichnet, dass** das medizinische Lignin in Form eines Pulvers oder Granulats vorliegt.

4. Zusammensetzung zur Verwendung nach einem der Ansprüche 1 bis 3, **dadurch gekennzeichnet, dass** die Zusammensetzung hydriert wird und dass die getrockneten Hefezellen ihre Lebensfähigkeit wiedererlangen und mit der Produktion von Wirkstoffen beginnen.

5. Zusammensetzung zur Verwendung nach einem der Ansprüche 1 bis 4, **dadurch gekennzeichnet, dass** sie für die Behandlung von Reisediarrhö verwendet wird.

6. Verwendung einer Zusammensetzung, die hydrolysiertes medizinisches Lignin und lebende Zellen des Hefestamms *Saccharomyces boulardii* in einer Menge von 10⁹ - 5 x 10¹⁰ lebenden Hefezellen pro Gramm hydrolysiertes Lignin umfasst für die Herstellung eines Arzneimittels zur Prophylaxe und Behandlung von infektiösen und nichtinfektiösen Diarrhöen.

## Revendications

1. Composition pharmaceutique pour une utilisation dans la prophylaxie et le traitement de diarrhées infectieuses et non infectieuses, comprenant de la lignine médicinale hydrolysée et des cellules vivantes de la souche de levure *Saccharomyces boulardii* prises à 10⁹-5 x 10¹⁰ cellules de levure vivantes pour 1 gramme de lignine hydrolysée.

2. Composition pour une utilisation selon la revendication 1, **caractérisée en ce qu'**elle est préparée par le mélange égal de poudre lyophilisée de cellules de levure *Saccharomyces boulardii* avec la poudre de lignine médicinale.

3. Composition pour une utilisation selon l'une quelconque des revendications 1 ou 2, **caractérisée en ce que** la lignine médicinale est présente sous la forme de poudre ou de granulés.

4. Composition pour une utilisation selon l'une quelconque des revendications 1 à 3, **caractérisée en ce que** ladite composition est hydratée et que les cellules de levure séchées récupèrent leur viabilité et commencent à produire des substances actives.

5. Composition pour une utilisation selon l'une quelconque des revendications 1 à 4, **caractérisée en ce qu'**elle est utilisée pour le traitement de la diarrhée des voyageurs.

6. Utilisation d'une composition comprenant de la lignine médicinale hydrolysée et des cellules vivantes de la souche de levure *Saccharomyces boulardii* prises à 10⁹-5 x 10¹⁰ cellules de levure vivantes pour 1 gramme de lignine hydrolysée, pour l'élaboration d'un médicament destiné à la prophylaxie et au traitement de diarrhées infectieuses et non infectieuses.
